# EUROPÄISCHE PATENTANMELDUNG

(11) **EP 2 641 892 A1**
(43) Veröffentlichungstag der Anmeldung: **25.09.2013**
(21) Anmeldenummer: 12160920.0
(22) Anmeldetag: 23.03.2012
(51) Int. Cl.: C07C 209/36, C07C 209/84, C07C 211/46

(54) **Verfahren zur Reinigung von Anilin aus Gasphasenhydrierungen**

(71) Anmelder: Bayer MaterialScience AG, 51373 Leverkusen (DE)
(72) Erfinder: Merkel, Michael, 40223 Düsseldorf (DE); Lehner, Peter, 77523-0000 Baytown, TX (US); Mahr, Bastian, 50735 Köln (DE); Moussa, Salah, Amgad, 51061 Köln (DE)

(57) **Zusammenfassung**

Die Erfindung betrifft ein Verfahren zur Reinigung von durch Gasphasenhydrierung von Nitrobenzol hergestelltem Anilin durch fraktionierende Kondensation des gasförmig anfallenden rohen Reaktionsproduktes in n Kondensationsstufen mit sukzessive sinkender Kondensationstemperatur, Destillation eines Teils oder aller der in den Kondensationsstufen 1 bis (n-1) erhaltenen Kondensate (der Partialkondensate PKⁱ) unter Erhalt eines destillierten Produkts PKD, Vereinigung des destillierten Kondensats PKD mit mindestens, bevorzugt ausschließlich, dem organischen Anteil des nten Kondensats (des Totalkondensats TK) und, sofern vorhanden, mit wenigstens einem Teil der verbliebenen nicht destillierten Partialkondensate PKⁱ, und Extraktion der so gewonnen Produktmischung mit wässriger Baselösung.

## Beschreibung

Die Erfindung betrifft ein Verfahren zur Reinigung von durch Gasphasenhydrierung von Nitrobenzol hergestelltem Anilin durch fraktionierende Kondensation des gasförmig anfallenden rohen Reaktionsproduktes in n Kondensationsstufen mit sukzessive sinkender Kondensationstemperatur, Destillation eines Teils oder aller der in den Kondensationsstufen 1 bis (n-1) erhaltenen Kondensate (der Partialkondensate PKⁱ) unter Erhalt eines destillierten Produkts PKD, Vereinigung des destillierten Kondensats PKD mit mindestens, bevorzugt ausschließlich, dem organischen Anteil des nten Kondensats (des Totalkondensats TK) und, sofern vorhanden, mit wenigstens einem Teil der verbliebenen nicht destillierten Partialkondensate PKⁱ, und Extraktion der so gewonnen Produktmischung mit wässriger Baselösung.

Aromatische Amine sind wichtige Zwischenprodukte, welche preiswert und in großen Mengen zur Verfügung stehen müssen. Anilin, ein technisch besonders bedeutsames aromatisches Amin, kann hervorragend nach dem erfindungsgemäßen Verfahren gereinigt werden. Anilin ist ein wichtiges Zwischenprodukt bei der Herstellung von Di- und Polyisocyanaten der Diphenylmethanreihe (MDI) und wird im großtechnischen Maßstab in der Regel durch katalytische Hydrierung von Nitrobenzol hergestellt. Hierfür müssen Anlagen mit sehr großen Kapazitäten gebaut werden, um den enormen weltweiten Bedarf decken zu können. Bevorzugt wird die Hydrierung von Nitrobenzol in der Gasphase an ortsfesten, heterogenen Trägerkatalysatoren, wie z. B. Pd auf Aluminiumoxid- oder Kohleträgern, in Festbettreaktoren bei einem absoluten Druck von 2 - 50 bar und einer Temperatur im Bereich von 250 - 500 °C unter adiabatischen Bedingungen in Kreisgasfahrweise durchgeführt, siehe EP-A-0 696 573, EP-A-0 696 574 und EP-A-1 882 681. **"Kreisgasfahrweise" bedeutet, dass die im rohen Reaktionsprodukt enthaltenen** nichtkondensierbaren Gase (also im Wesentlichen während der Hydrierung nicht umgesetzter Wasserstoff und ggf. zugesetzte oder durch Nebenreaktionen entstehende Inertgase) ggf. mit Ausnahme geringer, zum Konstanthalten der Konzentrationen weiterer gasförmiger Komponenten des Kreisgases - wie etwa von auf dem Katalysator durch Desaminierungsreaktionen gebildetes Ammoniak - abgezweigter Mengen, in die Reaktion zurückgeführt werden.

Bei der Herstellung von Anilin durch Hydrierung von Nitrobenzol werden neben dem Zielprodukt auch Wasser und organische Nebenkomponenten gebildet. Darüber hinaus können je nach Produktionsverfahren und Betriebszustand auch noch Anteile an nicht umgesetztem Nitrobenzol enthalten sein. Diese organischen Nebenkomponenten sowie ggf. nichtumgesetztes Nitrobenzol müssen vor einer weiteren Verwendung des Anilins bis auf Restgehalte von wenigen ppm abgetrennt werden. Die organischen Nebenkomponenten sowie ggf. nichtumgesetztes Nitrobenzol können in zwei Gruppen unterteilt werden: a) die Gruppe der "Leichtsieder", d. h. Verbindungen bzw. azeotrop siedende Gemische von Einzelkomponenten mit Siedepunkten unter denen von Anilin (Sdp. = 184 °C), und b) die Gruppe der "Schwersieder", d. h. Verbindungen bzw. azeotrop siedende Gemische von Einzelkomponenten mit Siedepunkten über denen von Anilin. Nitrobenzol (Sdp. = 211 °C) gehört demnach zur Gruppe der Schwersieder. Aufgrund des dem Anilin ähnlichen Siedepunkts auf destillativem Weg nur schwer abzutrennen ist Phenol (siehe z. B. EP-A-1 670 747), das in technischen Hydrierungen von Nitrobenzol stets als Nebenprodukt auftritt.

Ein roher Produktstrom einer Gasphasenhydrierung von Nitrobenzol besteht also in der Regel aus
(1) Anilin,
(2) Prozesswasser (d. i. die Summe aus in der Reaktion gebildetem und ggf. im Eduktgasstrom enthaltenen Wasser),
(3) (unter üblichen technischen Bedingungen der Anilinaufarbeitung) nichtkondensierbaren Gasen (Überschusswasserstoff - ggf. enthaltend gasförmige Verunreinigungen wie bspw. Methan und ggf. zugesetzte Inertgase, z. B. zur Selektivitätsverbesserung zugesetzter Stickstoff, vgl. EP-A-1 882 681, und ggf. gasförmige Nebenprodukte, z. B. Ammoniak aus Desaminierungsreaktionen),
(4) Leichtsiedern und
(5) Schwersiedern (die ggf. auch Anteile an nichtumgesetztem Nitrobenzol enthalten können). (1), (2), (4) und (5) werden nachfolgend auch als *"kondensierbare Bestandteile"* zusammengefasst.

Es ist Stand der Technik, das Anilin destillativ von allen Nebenkomponenten zu befreien. Aufgrund der schwersiedenden Anteile von Rohanilin (z. B. Diphenylamin mit Sdp. = 302 °C), muss hierzu das gesamte Anilin je nach Rücklaufverhältnis in der Destillation mindestens einmal verdampft und wieder kondensiert werden, so dass der Destillationsprozess hohe Energiekosten verursacht.

Eine besondere Schwierigkeit stellt die Abtrennung von solchen Nebenkomponenten dar, deren Siedepunkte denen des Anilins sehr ähnlich sind, weil hier der destillative Aufwand erheblich ist. In diesem Zusammenhang stellt insbesondere die Abtrennung von Phenol (Sdp. = 182 °C) eine große Herausforderung für die Destillationstechnik dar, was sich in der Verwendung langer Destillationskolonnen mit großer Trennstufenzahl und hohen Rücklaufverhältnissen mit entsprechend hohem Investitions- und Energieaufwand widerspiegelt. Verbindungen mit phenolischen Hydroxygruppen, d. h. Verbindungen, die mindestens eine Hydroxygruppe (-OH) direkt an einem aromatischen Ring tragen, können generell bei der Aufarbeitung von Anilin problematisch sein. Neben dem bereits erwähnten Phenol sind insbesondere die verschiedenen Aminophenole zu nennen. Diese sind zwar aufgrund des höheren Siedepunkts destillativ leichter abzutrennen, können aber sowohl einen Viskositätsanstieg im Kolonnen-Sumpf hervorrufen als auch Ablagerungen im Destillationsapparat verursachen, wenn Basen, wie z. B. Alkalimetallhydroxid im Destillationsapparat zugegen sind, um die Phenolabtrennung zu optimieren.

Die Reinigung von Anilin ist daher nicht trivial und besitzt große industrielle Bedeutung. Viele Ansätze widmen sich insbesondere der erwähnten Problematik im Zusammenhang mit Verbindungen mit phenolischen Hydroxygruppen. Der Lösungsansatz besteht darin, die Verbindungen mit phenolischen Hydroxygruppen durch Reaktion mit geeigneten Basen in die entsprechenden Salze zu überführen, welche sich als nichtflüchtige Verbindungen wesentlich leichter abtrennen lassen.

So offenbaren JP-A-49-035341**,** EP-A-1 845 079**,** EP-A-2 028 176 und EP-A-1 670 747 Verfahren, bei denen ein aromatisches Amin *in Gegenwart einer Base* destilliert wird. Bei dieser Verfahrensweise müssen Probleme durch Feststoffabscheidung, Fouling und/oder starken Viskositätsanstieg bei der Destillation durch aufwändige und/oder teure Maßnahmen verhindert werden.

Als Alternative zur Entfernung von Verbindungen mit phenolischen Hydroxygruppen aus Anilin während der Destillation beschreibt JP-A-08-295654 eine Extraktion mit verdünnter wässriger Alkalihydroxidlösung und anschließender Destillation der organischen Phase. Nachteilig bei diesem Verfahren sind der hohe NaOH-Verbrauch und das Anfallen - infolge der geringen Konzentration der Alkalihydroxid-Lösungen - sehr großer Mengen alkaliphenolathaltigen Abwassers, zusätzlich zum hohen Energieverbrauch in der Destillation.

EP-A-1 845 080 beschreibt ein Verfahren zur Reinigung von Anilin durch Extraktion mit wässriger Alkalimetallhydroxid-Lösung einer Konzentration > 0,7 Massen-%, wobei Konzentration und Temperatur so eingestellt werden, dass die wässrige Phase bei der anschließenden Phasentrennung immer die untere Phase darstellt. Optional kann zum Erreichen einer gewünschten Produktqualität wiederum das gesamte Rohprodukt vor oder nach der Extraktion destilliert werden.

JP-A-2007217405 beschreibt ein Verfahren, in dem das phenolhaltige Anilin mindestens zweimal mit wässriger Alkalimetallhydroxidlösung so in Kontakt gebracht wird, dass die Konzentration an Alkalimetallhydroxid in der wässrigen Phase zwischen 0,1 Massen-% und 0,7 Massen-% liegt. Anschließend erfolgt eine Trennung von wässriger und organischer Phase und Destillation der organischen Phase.

Ganz allgemein mit der Verbesserung der Anilinaufarbeitung befasst sich JP-A-2005 350388. Es wird ein Verfahren beschrieben, bei dem ein Teil des Sumpfes der Anilin-Destillationskolonne aus dieser abgeführt und separat, d. h. in einem vom eigentlichen Verdampfer der Kolonne verschiedenen zweiten Verdampfer, in die Gasphase überführt wird. Die so erhaltene Gasphase wird in die Reinanilinkolonne zurückgeführt; nicht verdampfbare Schwersiederanteile werden abgetrennt. Nachteilig bei diesem Verfahren ist, dass Leichtsieder und Wasser vor der eigentlichen Anilin-Destillationskolonne in einem apparativ aufwändigen Verfahren in einer Entwässerungskolonne separat durch eine zusätzliche Destillation abgetrennt werden müssen.

Keine dieser bisher genannten Druckschriften geht darauf ein, wie es erreicht werden kann, den Anteil des Anilins, der in einem Destillationsprozess verdampft und erneut kondensiert werden muss, zu verringern. Stammt das zu reinigende Anilin aus einem Gasphasenprozess, so durchläuft es nach dem Stand der Technik sogar zwei Kondensationen: Zunächst wird das gasförmig anfallende Reaktionsprodukt möglichst vollständig kondensiert, die wässrige Phase abgetrennt und die erhaltene organische Phase destilliert, d. h. das gewünschte Produkt wird (i) kondensiert, (ii) verdampft und (iii) wieder kondensiert, was energetisch und apparativ sehr aufwändig ist und zu thermischen Belastungen des Anilins führt.

Lediglich **in der noch nicht veröffentlichten Anmeldung mit dem Aktenzeichen** PCT/EP2011/068122 wird auf diese Problematik eingegangen. Dort ist beschrieben, dass das Anilin aus einem Gasphasenprozess fraktionierend kondensiert wird, wobei der aus der Partialkondensation (PK) stammende Produktstrom in den unteren Teil der Destillationskolonne zwischen dem untersten Abtriebsteil und der darauf folgenden Sektion und der aus der Totalkondensation (TK) stammende Produktstrom in den Kopf der Destillationskolonne oberhalb des obersten Verstärkungsteils eingeleitet werden. Destilliertes Anilin wird in einem Seitenstrom zwischen unterstem Abtriebsteil und oberstem Verstärkungsteil der Destillationskolonne entnommen. Durch diese Ausführungsform wird erreicht, dass der aus der Totalkondensation stammende Produktstrom nicht mehr verdampft werden muss, sondern direkt in der Destillationskolonne durch Strippung von Leichtsiedern befreit wird.

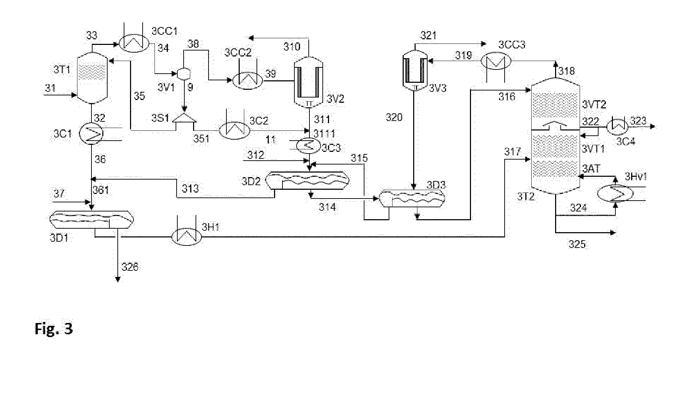

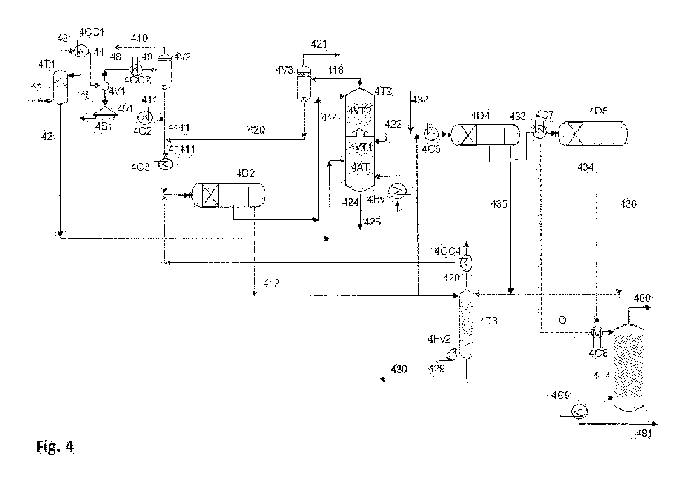

Da die Entfernung von Phenol auf destillativem Weg problematisch ist, erfordert das beschriebene Verfahren gleich mehrere Extraktionen, nämlich individuell für jeden Produktstrom (Ausführung gemäß Fig. 3 in PCT/EP2011/068122). Soll darüber hinaus verhindert werden, dass Salze in den Destillationsapparat gelangen, muss jeder dieser Extraktionen zusätzlich eine weitere Extraktionsstufe nachgeschaltet werden, in der der Produktstrom mit Wasser gewaschen wird. Alternativ kann die Extraktion auch nach der Destillation erfolgen (Ausführung gemäß Fig. 4 in PCT/EP2011/068122), jedoch wird dabei bereits gestripptes Produkt wieder mit Wasser gesättigt und muss gegebenenfalls einer erneuten Strippung unterzogen werden. Nachteilig ist außerdem die starke Kopplung zwischen den einzelnen Kondensaten, die dadurch entsteht, dass in der Destillationsapparatur der aus PK aufsteigende *Produkt*dampf genutzt wird, um den aus TK stammenden Produktstrom zu strippen. Je nach Produktqualität führt dies zu einem erhöhten Energieaufwand, weil z. B. trotz niedrigem Schwersiedergehalt im Produktstrom aus PK ein großer Anteil des Gesamt-Produkts als PK in den unteren Teil der Kolonne gefahren und verdampft werden muss, um eine ausreichende Strippung des Produktstroms aus TK zu gewährleisten. Mit anderen Worten, die Auftrennung des Gesamt-Rohprodukts auf TK und PK kann nicht immer so erfolgen, wie es im Sinne einer wirtschaftlich optimalen Aufarbeitung wünschenswert wäre, sondern unterliegt auch gewissen Zwängen, die sich aus der Art der Destillation ergeben. Es bestehen also unbefriedigende Kopplungsmechanismen bei Aufteilung des Gesamt-Rohprodukts in der fraktionierenden Kondensation in einzelne Teilströme und deren anschließende Aufarbeitung.

Es bestand daher ein Bedarf an einem Verfahren zur Reinigung von aus Gasphasenhydrierungen stammendem Anilin, bei dem nur ein möglichst geringer Anteil des Anilins selbst erneut wieder verdampft und kondensiert werden muss und bei dem die Abtrennung von Verbindungen mit phenolischen Hydroxygruppen möglichst effektiv mit möglichst geringen Verlusten an wertvollem Anilin erreicht wird. Insbesondere sollten auch unerwünschte Kopplungsmechanismen bei der Aufteilung des Gesamt-Rohprodukts in der fraktionierenden Kondensation in einzelne Teilströme und deren anschließende Aufarbeitung auf ein Minimum reduziert werden.

Dem Vorstehend Gesagten Rechnung tragend ist ein Gegenstand der vorliegenden Erfindung ein Verfahren zur Herstellung von Anilin umfassend die folgenden Schritte:
(i) Gasphasenhydrierung von Nitrobenzol in Gegenwart eines Katalysators,
(ii) fraktionierende Kondensation des in (i) anfallenden gasförmigen Rohproduktes in n Kondensationsstufen, wobei n eine natürliche Zahl von 2 bis 8, bevorzugt von 3 bis 4 und besonders bevorzugt gleich 3 ist, mit sukzessive sinkender Kondensationstemperatur, wobei in der ersten bis (n-1)ten Kondensationsstufe jeweils ein flüssiges Partialkondensat PKⁱ (PK¹, PK², ... PKⁿ⁻¹) und in der nten Kondensationsstufe ein flüssiges Totalkondensat (TK) erhalten wird,
(iii)
   falls (a) n = 2 ist, Destillation des einen in (ii) erhaltenen flüssigen Partialkondensats PK¹,
   falls (b) n ≥ 3 ist, Destillation eines Teils der in (ii) erhaltenen flüssigen Partialkondensate PKⁱ, bevorzugt nur des ersten Partialkondensats PK¹,
   wobei ein Destillat PKD, bevorzugt nur PK¹D (also das Destillat von PK¹), erhalten wird,
(iv) Vereinigung
   (a) sofern vorhanden, wenigstens eines Teils, bevorzugt aller, der in (ii) erhaltenen und nicht in (iii) destillierten Partialkondensate PKⁱ,
   (b) des in (iii) erhaltenen Destillats PKD und
   (c) mindestens, bevorzugt ausschließlich, des organischen Anteils des Totalkondensats TK, Extraktion der so gewonnenen Produktmischung mit wässriger Baselösung und Trennung des so erhaltenen Gemischs in eine wässrige Phase und in eine organische, Anilin umfassende Phase.

Nachstehend wird die Erfindung im Detail erläutert. Dabei sind verschiedene Ausführungsformen frei miteinander kombinierbar, sofern sich für den Fachmann aus dem Kontext nicht eindeutig das Gegenteil ergibt.

Die *Gasphasenhydrierung von Nitrobenzol* in **Schritt (i)** erfolgt kontinuierlich und kann nach jedem aus dem Stand der Technik bekannten Verfahren und an allen aus dem Stand der Technik bekannten Katalysatoren erfolgen. Sowohl isotherme als auch adiabatische Prozesse wurden beschrieben. Besonders bevorzugt sind die in EP-A-0 696 573, EP-A-0 696 574 und EP-A-1 882 681 (adiabatische Prozessführung) sowie die in GB-A-1452466 und EP-A-0 944 578 (isotherme Prozessführung) beschriebenen Verfahren. Die adiabatischen Prozesse sind besonders bevorzugt, unter ihnen ist der in EP-A-1 882 681 beschriebene der am stärksten bevorzugte Prozess. Neben den genannten Verfahren mit stationären Katalysatorbetten sind auch solche mit fluidisierten Katalysatorbetten z. B. in DE-1114820-B, DE-1133394-B oder WO-2008-034 770 A1 beschrieben. Auch diese können im erfindungsgemäßen Schritt (i) angewandt werden. Bevorzugt sind jedoch stationäre Katalysatorbetten. Besonders bevorzugt sind die in EP-A-1 882 681 (Absätze [0035] bis [0050]) beschriebenen Katalysatoren.

Allen Verfahren ist gemein, dass das Rohprodukt gasförmig anfällt.

Dieses in Schritt (i) erhaltene gasförmige Rohprodukt wird nun in **Schritt (ii)** fraktionierend kondensiert, d. h. das Rohprodukt aus (i) wird zunächst in einer ersten Kondensationsstufe soweit gekühlt, dass nur *ein Teil* der kondensierbaren Bestandteile verflüssigt wird (nämlich neben Anilin überwiegend diejenigen mit dem höchsten Siedepunkt, also Verbindungen aus der Gruppe der Schwersieder). Es wird also in der ersten Kondensationsstufe nur ein *Partial*kondensat (PK¹) erhalten. Die Gasphase der ersten Kondensationsstufe wird der zweiten Kondensationsstufe zugeführt usw. Erst in der letzten (nten) Kondensationsstufe wird die eintretende Gasphase soweit abgekühlt, dass die kondensierbaren Bestandteile möglichst vollständig kondensieren (*Total*kondensation, TK). Da der Kühlung aufgrund wirtschaftlicher Randbedingungen Grenzen gesetzt sind, kann nicht ausgeschlossen werden, dass geringe Mengen an kondensierbaren Bestandteilen in der Gasphase verbleiben. Bevorzugt wird das Prozesswasser überwiegend erst in der Totalkondensationsstufe (der nten Kondensationsstufe) verflüssigt, sodass alle zuvor erhaltenen Partialkondensate bevorzugt einphasig sind. Zwischen der ersten und der nten Kondensationsstufe befinden sich 0 bis 6, bevorzugt 1 bis 2 weitere Partialkondensationsstufen PK², PK³ usw. Besonders bevorzugt umfasst Schritt (ii) des erfindungsgemäßen Verfahrens genau drei Kondensationsstufen mit drei Kondensatströmen, PK¹, PK² und TK.

Als Apparate für die Durchführung der fraktionierenden Kondensation kommen grundsätzlich alle dem Fachmann zum Zwecke der Kondensation von Gasen bekannten Vorrichtungen in Frage. Als Bespiele seien Luftkühler genannt oder Wärmeaustauscher wie z. B. Rohrbündelwärmeaustauscher oder Plattenwärmeaustauscher, bei denen die abgegebene Wärme zum Aufheizen eines anderen gasförmigen oder flüssigen Stromes genutzt werden kann.

Bevorzugt wird die erste Kondensationsstufe bei einer Temperatur T¹ von 100 °C bis 200 °C, besonders bevorzugt von 100 °C bis 150 °C, die nte Kondensationsstufe bei einer Temperatur Tⁿ von 15 °C bis 90 °C, bevorzugt von 20 °C bis 80 °C, besonders bevorzugt von 40 °C bis 70 °C, betrieben, und die Temperatur jeder weiteren Kondensationsstufe, sofern vorhanden, liegt bevorzugt jeweils um 1 K bis 100 K, besonders bevorzugt jeweils um 2 K bis 50 K, ganz besonders bevorzugt um 5 K bis 40 K unter der vorangehenden Kondensationsstufe, wobei die Temperaturstufen nicht gleichverteilt sein müssen. Im Falle von mehr als drei Kondensationsstufen sind bevorzugt die ersten und letzten Temperaturschritte größer als die dazwischenliegenden.

Es kann auch vorteilhaft sein, im Laufe eines Reaktionszyklus der Gasphasenhydrierung die Kondensationstemperaturen, Rücklaufverhältnisse oder die Anzahl der genutzten Kondensationsstufen zu variieren, da die Selektivität der Hydrierung zu Beginn eines Reaktionszyklus der Gasphasenhydrierung in der Regel schlechter ist als zu einem späteren Zeitpunkt (nachdem alle Betriebsparamater der Gasphasenhydrierung auf die optimalen Bedingungen eingestellt sind und der Katalysator seinen optimalen Wirkungsbereich hinsichtlich der Selektivität erreicht hat) eines Reaktionszyklus. In einer solchen Verfahrensweise würde z. B. mit sinkendem Schwersiedergehalt des Rohproduktstroms die Kondensationstemperatur in den ersten Kondensationsstufen erhöht und/oder der Rücklauf in den ersten Wäscher reduziert, um den zu destillierenden Anteil des Anilins und damit den Energieaufwand zu reduzieren. Wird während der Reaktionsphase der Druck im Reaktionssystem variiert (z. B. erhöht, vgl. EP 6 965 741 B1 Abschnitte [0056] bis [0057]), so ist es vorteilhaft, auch die Kondensationstemperaturen entsprechend der physikalischen Gesetzmäßigkeiten anzupassen, d. h. bei Erhöhung des Drucks zu erhöhen und bei Erniedrigung des Drucks zu senken.

In **Schritt (iii)** wird, falls n ≥ 3 ist, ein Teil der in Schritt (ii) erhaltenen Partialkondensate PKⁱ, bevorzugt nur das erste Partialkondensat PK¹, einer Destillation unterworfen, bei der ein Destillat PKD, bevorzugt nur PK¹D, erhalten wird, welches gegenüber den Ausgangs-Partialkondensaten (d. h. in der bevorzugten Ausführungsform gegenüber dem ersten Partialkondensat PK¹) an Anilin angereichert ist. Hierzu werden bevorzugt die zu destillierenden Partialkondensate PKⁱ vereinigt und gemeinsam destilliert. Grundsätzlich möglich, jedoch aufgrund des großen apparativen Aufwands keinesfalls bevorzugt, wäre es auch, jedes Partialkondensat einzeln zu destillieren und die einzelnen Destillate PKⁱD zu PKD zu vereinigen. Falls n = 2 ist, gibt es nur ein Partialkondensat. Dieses wird in jedem Fall einer Destillation unterworfen.

Als Destillationsapparatur eignen sich alle dem Fachmann bekannten Vorrichtungen. Das Destillat PKD wird entweder als Seitenstrom oder am Kopf, bevorzugt am Kopf der Destillationsapparatur entnommen.

Das letzte (nte) Kondensat (TK) enthält den Hauttpteil des Prozesswassers und wird vor der weiteren Verarbeitung bevorzugt einer Phasentrennung in eine anilinreiche organische Phase (TKO) und eine wässrige Phase (TKW) unterzogen. Dies geschieht vor Schritt (iv) in dem Fachmann bekannten Phasentrennbehältern (z. B. Flüssig-flüssig-Abscheidern). Auf diese Weise wird gewährleistet, dass die Phasentrennung an dieser Stelle nicht durch Salze gestört wird, und es wird ein salzfreier, wasserreicher Strom erhalten (TKW). In dieser bevorzugten Ausführungsform wird in Schritt (iv) nur (c) TKO mit (a) sofern vorhanden (im Fall n = 2 gibt es keine nicht destillierten Partialkondensate), wenigstens einem Teil, bevorzugt allen, der in (ii) erhaltenen und nicht in (iii) destillierten Partialkondensate PKⁱ und (b) dem in (iii) erhaltenen Destillat PKD vereinigt und die so gewonnene Produktmischung mit wässriger Baselösung extrahiert. Die Vermischung des gesamten Totalkondensats, also einschließlich des Prozesswassers, mit (a) (sofern vorhanden) und (b) ist grundsätzlich möglich, jedoch nicht bevorzugt, da in diesem Fall zur Reduktion der Salzfracht des Produkts in einem weiteren Extraktionsschritt ein zusätzlicher wässriger Strom eingeführt werden müsste, der wiederum mit Produkt gesättigt würde, so dass entweder Produktverluste entstehen oder zusätzlicher Aufwand für die Rückgewinnung des Produkts betrieben werden muss. Als Basen werden dabei bevorzugt wässrige Lösungen von Alkali- oder Erdalkalimetallhydroxiden (oder Mischungen aus beiden) verwendet. Dabei ist zu beachten, dass die Konzentration der Basenlösung so gewählt wird, dass eine Phasentrennung zuverlässig stattfindet, d. h. dass einerseits hinreichende Volumina von beiden Phasen (wässrig und organisch) vorliegen und andererseits ein hinreichender Dichteunterschied bei der gewählten Extraktionstemperatur vorliegt, so dass es im Betrieb nicht zu einer ungewollten Phasenumkehr kommt. Dies kann entweder durch eine geringe oder eine höhere Basenkonzentration erreicht werden (vgl. JP-A-08-295654 bzw. EP-A-1 845 080). Das in der Extraktion erhaltene Gemisch wird in eine wässrige Phase und in eine organische, Anilin umfassende Phase getrennt. Letztere kann abhängig von den genauen Bedingungen bereits ausreichend rein sein, um der angestrebten Verwendung des Anilins zugeführt zu werden. Zur Reduzierung des Salzgehalts kann es aber auch angebracht sein, in einem zusätzlichen Schritt (v) die in (iv) erhaltene organische, Anilin umfassende Phase mit einem mindestens 85 Massen-% Wasser umfassenden Strom, bezogen auf die Gesamtmasse dieses Stroms, zu waschen. Bevorzugt ist das Verhältnis der Gesamtkonzentrationen an Salzen in diesem wässrigen Strom und der Anilin umfassenden Phase kleiner als 10 : 1, bevorzugt kleiner als 1 : 1, besonders bevorzugt kleiner als 0,1 : 1. Der mindestens 85 Massen-% Wasser umfassende Strom kann z B. durch Kondensation von in der Produktion anfallenden wasserhaltigen Dämpfen erzeugt werden, so dass sehr niedrige Salzgehalte erreicht werden. In der bevorzugten Ausführungsform mit einer Phasentrennung von TK vor Schritt (iv) wird besonders bevorzugt ein Teil oder die Gesamtheit der in der Herstellung von TKO anfallenden wässrigen Phase TKW als mindestens 85 Massen-% Wasser umfassender Strom eingesetzt.

Bevorzugt wird die Anilin umfassende Phase - entweder direkt wie in (iv) erhalten oder nach Waschung mit dem genannten mindestens 85 Massen-% Wasser umfassenden Strom - einer Strippung unterzogen. Hierzu ist dem Fachmann eine Vielzahl von Ausführungsformen bekannt. Bevorzugt erfolgt die Strippung in einer Destillationsapparatur, wobei die Anilin umfassende Phase gegebenenfalls vorerwärmt und dann am Kopf der Apparatur aufgegeben wird. Am unteren Ende der Apparatur befindet sich ein mit Wasserdampf betriebener Umlaufverdampfer. Das an Wasser und Leichtsiedern abgereicherte Anilin wird am Boden der Apparatur entnommen, Wasser und Leichtsieder werden als Brüden über Kopf abgenommen. Bevorzugt wird aus den Brüden mitgeschlepptes Anilin kondensiert und dem Totalkondensatstrom zugeführt.

Auch alle mit organischen Phasen kontaktierten Wasserphasen des Verfahrens, also z. B. die in (iv) erhaltene Wasserphase und die in in der bevorzugten Ausführungsform vor Schritt (iv) aus TK abgetrennte wässrige Phase (TKW) oder davon abgezweigte Teilströme, können einer mit Wasserdampf beheizten Strippung unterworfen werden, um Anilin als Azeotrop mit Wasser zurückzugewinnen, das dann wiederverwendet werden kann, z. B. **indemes** dem Totalkondensatstrom TK zugeführt wird. Soll eine solche Strippung erfolgen, erweist es sich als besonders vorteilhaft, die zu strippenden wässrigen Ströme zunächst zu vereinen und das Abwasser vor Eintritt in den Stripper durch Wärmeaustausch mit den Anilin-Brüden aus der Destillation vorzuerwärmen.

Das Verfahren wird im Folgenden mit Hilfe der beigefügten Zeichnungen anhand der besonders bevorzugten Ausführungsform, welche die Bildung von genau drei Kondensatströmen (PK¹, PK² und TK), eine Trennung von TK in eine anilinreiche organische Phase (TKO) und eine wasserreiche Phase (TKW) sowie eine zweistufige Extraktion mit anschließender Strippung der gewaschenen organischen Phase zum Gegenstand hat, näher erläutert. Es ist für den Fachmann ein Leichtes, auf Basis dieser Beschreibung das Verfahren mit drei Kondensationsstufen so zu modifizieren, dass es mehr als drei Kondensatströme umfasst, etwa indem der möglichst vollständigen letzten Kondensationsstufe (TK) zusätzliche partielle Kondensationsstufen PK³, PK⁴ usw. vorgeschaltet sind.

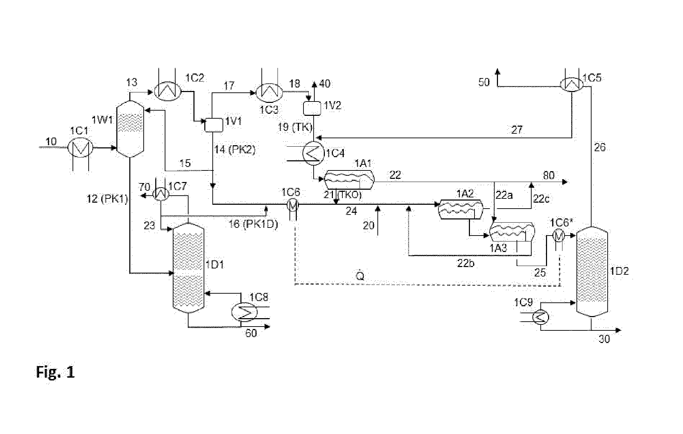

Eine bevorzugte Ausführungsform des erfindungsgemäßen Verfahrens wird im Folgenden mit Hilfe von Fig. 1 näher erläutert.

Es bedeuten:

**Tabelle 1: Bezugszeichen der Fig. 1.**

| **Stoffstrom** | **Bedeutung** | **Apparat** | **Bedeutung** |
|---|---|---|---|
| **10** | Rohanilin aus der Hydrierung | **1A1** | Flüssig-Flüssig-Abscheider |
| **20** | Natronlauge | **1A2** | Flüssig-Flüssig-Abscheider |
| **30** | Reinanilin | **1A3** | Flüssig-Flüssig-Abscheider |
| **40** | Überschusswasserstoff (wird zurück zum Kreisgas geführt, nicht gezeigt) | **1C1** | Kreisgaskühler |
| **50** | Abgas | **1C2** | Kondensator |
| **60** | Schwersieder-Purge aus dem Sumpf von **1D1** | **1C3** | Kondensator |
| **70** | Abgas aus Schwersiederkolonne | **1C4** | Wärmetauscher |
| **80** | Abwasser | **1C5** | Kondensator |
| **12** | Flüssige Phase aus **1W1,** erstes Partialkondensat (PK¹) | **1C6, 1C6*** | Wärmeaustauscher |
| **13** | Gasstrom aus **1W1** | **1C7** | Kondensator |
| **14** | Flüssige Phase aus **1V1,** zweites Partialkondensat (PK²) | **1C8** | Verdampfer |
| **15** | Teilstrom von 14 als Rücklauf in Wäscher **1W1** | **1C9** | Verdampfer |
| **16** | Kopfprodukt aus **1D1** (PK¹D) | **1D1** | Schwersiederkolonne |
| **17** | Gasstrom aus **1V1** | **1D2** | Anilin-Stripper |
| **18** | Abgekühlter Strom aus **1C3** | **1V1** | Gas-Flüssig-Abscheider |
| **19** | Flüssige Phase aus **1V2,** Totalkondensat (TK) | **1V2** | Gas-Flüssig-Abscheider |
| **21** | Organische Phase aus **1A1,** (TKO) | **1W1** | Wäscher zum Zurückhalten von Schwersiedern |
| **22** | Wässrige Phase aus **1A1** | | |
| **22a** | Teilstrom von 22 zum **1A3** | | |
| **22b** | Wässrige Phase aus **1A3** | | |
| **22c** | Wässrige Phase aus **1A2** | | |
| **23** | Rücklauf in die Schwersiederkolonne | | |
| **24** | Vereinigter Rohproduktstrom | | |
| **25** | Organische Phase aus **1A3** | | |
| **26** | Gasphase aus **1D2** | | |
| **27** | Kondensat aus Strom 26 | | |
| **Q** | Wärmeintegration zwischen **1C6** und **1C6*** | | |

**D**as gasförmige Reaktionsprodukt, Strom **10,** bestehend aus Anilin, Prozesswasser, nichtkondensierbaren Gasen, Leichtsiedern und Schwersiedern, durchläuft die drei Kondensationsstufen unter einem absoluten Druck von 1,0 bar bis 50 bar, bevorzugt von 2,0 bar bis 20 bar und besonders bevorzugt von 2,0 bar bis 10 bar. Das Gasförmige Reaktionsprodukt wird zunächst in Wärmeaustauscher **1C1** auf 100 °C bis 200 °C, bevorzugt 100 °C bis 150 °C abgekühlt und in den Wäscher **1W1** geleitet. Am Kopf von **1W1** wird Strom **13** entnommen und im Kondensator **1C2** auf eine Temperatur von 60 °C bis 160 °C, bevorzugt von 80 °C bis 140 °C und besonders bevorzugt von 80 °C bis 110 °C abgekühlt (*zur Bildung von PK² führende Kondensation*)*.* Der so erhaltene Strom wird im Abscheider 1V1 in eine gasförmige und eine flüssige Phase **(PK²)** getrennt. Ein Teilstrom (Strom **15)** von **PK²** kann in den Wäscher **1W1** zurückgeleitet werden, um Schwersieder aus Strom **10** weitgehend zurückzuhalten und so für einen möglichst schwersiederfreien Strom **13** zu sorgen. Aus **1W1** wird auf diese Weise ein an Schwersiedern reiches, flüssiges Verfahrensprodukt abgezogen (Strom **12, PK¹**) **PK¹** enthält neben den Schwersiedern (bevorzugt 90 Massen-% bis 100 Massen-% der in Strom 10 enthaltenen Schwersieder) noch Anteile an Anilin (bevorzugt 0,1 Massen-% bis 35 Massen-% des in Strom **10** enthaltenen Anilins), an Leichtsiedern (bevorzugt <1 Massen-% der in Strom **10** enthaltenen Leichtsieder) sowie an Prozesswasser (bevorzugt < 5 Massen-% des in Strom **10** enthaltenen Prozesswassers).

Der so erhaltene Strom **12** wird in einen Destillationsapparat **1D1** geleitet und dort bei vermindertem Druck destilliert, wobei sich wiederum die Schwersieder im Sumpf der Kolonne anreichern und vom anilinreichen Kopfprodukt (**PK¹D**) abgetrennt werden.

Der aus **1V1** abgezogene gasförmige Strom **17** wird im Kondensator **1C3** teilweise kondensiert, sodass sich ein flüssiges Verfahrensprodukt (*zur Bildung von TK führende Kondensation*) neben einer verbleibenden Gasphase bildet. Die Kondensationstemperatur richtet sich nach den ökonomischen Randbedingungen: Eine zu hohe Temperatur führt zu unerwünschten Produktverlusten, eine zu geringe Temperatur zu unvertretbarem Energieaufwand für die Kondensation. Die tatsächlich gewählte Kondensationstemperatur stellt daher einen Kompromiss dar und beträgt bevorzugt von 15 °C bis 90 °C, besonders bevorzugt von 20 °C bis 80 °C und ganz besonders bevorzugt von 40 °C bis 70 °C. In diesem Verfahrensschritt kondensiert nicht nur Anilin, sondern es kondensieren auch Wasser und Leichtsieder, sowie ggf. geringe Anteile an mitgeschleppten Schwersiedern, aus.

Der aus **1C3** austretende Strom **18** wird in den Abscheider **1V2** geleitet, um die flüssige Phase von der gasförmigen Phase zu trennen. Die gasförmige Phase, die den aus der Reaktion stammenden Überschusswasserstoff enthält, wird bevorzugt als Kreisgas in die Nitroaromatenreduktion zurückgeführt (Strom **40).** Das als Strom **19** abgezogene flüssige Verfahrensprodukt **(TK)** wird, optional nach Durchlaufen eines weiteren Wärmetauschers, **1C4,** in einem Trennbehälter **(1A1)** in eine wässrige (Strom **22)** und eine organische Phase (Strom **21, TKO)** getrennt.

Die organischen Produktströme **14, 16** und **21 (PK², PK¹D** und **TKO)** werden vereinigt und mit einer Basenlösung, bevorzugt Alkalimetallhydroxid-Lösung und optional mit zusätzlichem Wasser versetzt.

Bevorzugt wird zunächst eine höher konzentrierte Base zugesetzt und die basenhaltige Mischung gründlich vermischt. Die Vermischung erfolgt mit einer dem Fachmann bekannten Mischreinrichtung, bevorzugt mittels einer Pumpe. Dann wird mit weiterem Wasser, bevorzugt einem Teil des von **TK** abgetrennten Prozesswassers, versetzt, und erneut gründlich, bevorzugt mittels einer Pumpe, vermischt, bevor in einem Trennbehälter (**1A2**) die wässrige von der organischen Phase getrennt wird. In einer besonders bevorzugten Ausführungsform des erfindungsgemäßen Verfahrens wird die aus **1A2** erhaltene organische Phase anschließend einem zweiten Extraktionsschritt **(1A3)** unterzogen. Dabei wird das von **TK** abgetrennte Prozesswasser zunächst verwendet, um die organische Phase aus der Phenol-Extraktion zu waschen und auf diese Weise in der Organik verbliebene Salzreste zu entfernen. Nach Phasentrennung kann das Wasser dann zur Extraktion der basenhaltigen Rohproduktmischung im ersten Extraktionsschritt **(1A2)** eingesetzt werden (Phenolatextraktion).

An dieser Stelle sei angemerkt, dass aus Gründen der Übersichtlichkeit die Flüssig-Flüssig-Abscheider in den hier enthaltenen Abbildungen stets so gezeichnet sind, dass die organische (anilinreiche) Phase sich unten absetzt, während die wässrige (wasserreiche) Phase die obere Phase bildet. Dies ist natürlich in der Praxis nicht immer der Fall. Vielmehr haben die jeweiligen Prozessparameter wie z. B. Temperatur und Salzgehalt einen Einfluss auf die Dichten der Flüssigkeiten und können deshalb auch zu einer umgekehrten Anordnung der Phasen führen. Dies ist für die Ausführbarkeit der Erfindung unerheblich, muss aber in Form einer entsprechend geänderten Verrohrung der Abscheider berücksichtigt werden.

Die organische Phase aus der zweiten Extraktionsstufe enthält neben Anilin noch Leichtsieder und Wasser. Um die Gehalte an diesen Nebenkomponenten auf ein spezifikationsgerechtes Niveau abzusenken, wird das Anilin am Kopf auf eine Strippkolonne **1D2** gegeben. Aus dem Kopfproduktstrom **26** kann ein Anilin und leichtsiedende Nebenkomponenten enthaltendes Kondensat **27** zurückgewonnen werden, das in die Phasentrennung **1A1** zurückgeführt wird. Anilin wird in einer für anschließende Prozesse geeigneten Qualität am Sumpf der Strippkolonne abgezogen. Um den Energieeinsatz im Verdampfer **1C9** zu reduzieren, kann in **1C6*** eine Vorwärmung des Kolonnenzuflusses **25** erfolgen, bevorzugt unter Ausnutzung der bei der Abkühlung in **1C6** abgeführten Wärme.

Falls erforderlich ist es ohne weiteren apparativen Aufwand möglich, ein nahezu wasserfreies Produkt **(30)** zu erhalten, indem die Verdampfungsrate in **1C9** erhöht wird. Für den Einsatz des Anilins in der Herstellung von Di- und Polyaminen der Diphenylmethanreihe (dem größten Anwendungsgebiet) ist dies normalerweise jedoch nicht erforderlich.

Die vereinigten Prozesswasserströme aus den Extraktionsstufen (Strom 80) können, soweit nicht an anderer Stelle im Prozess eingesetzt, in einer dem Fachmann bekannten Weise gestrippt werden (nicht gezeigt in Fig. 1) um den Prozesswasserstrom zu reinigen und Anilin zurückzugewinnen, z. B. indem ein Anilin-Wasser Gemisch als Azeotrop zurückgewonnen wird, das wiederum in den Trennbehälter **1A1** zugeführt wird. Auf diese Weise werden die Verluste an Anilin minimiert.

### Beispiele

In den folgenden Beispielen wird die Reinigung eines Rohanilins beschrieben, welches mit einem Massenstrom von 35 000 kg / h und einer Temperatur von 147 °C einer Produktionsanlage (Gasphasenhydrierung) entströmt und wie folgt zusammengesetzt ist:

**Tabelle 2: Zusammensetzung des zu reinigenden Rohanilinstromes.**

| **Komponente** | **Massenanteil in %** |
|---|---|
| Nicht kondensierbare Gase | 25,50 |
| Leichtsieder | 0,72 |
| Wasser | 28,20 |
| Anilin | 45,51 |
| Phenol | 0,02 |
| Schwersieder | 0,05 |

In allen Fällen wurde als wässrige Baselösung 32%ige Natronlauge eingesetzt, und zwar in knapp unterstöchiometrischer Menge in Bezug auf phenolische Hydroxygruppen.

### Beispiel 1 (Vergleichsbeisniel) ASPEN-Simulation

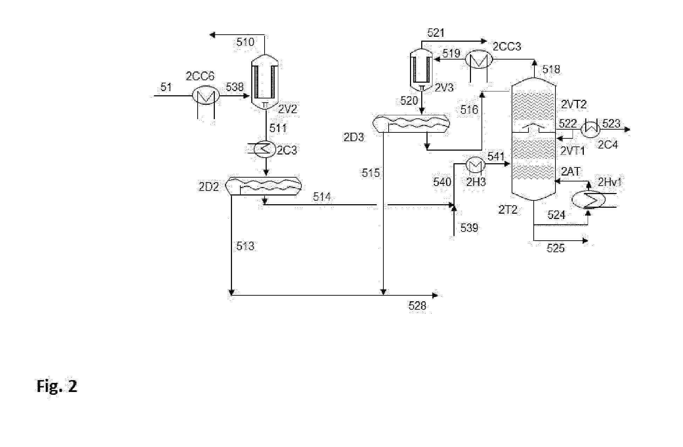

Fig. 2 zeigt den in diesem Beispiel angewandten Prozess. Es bedeuten:

**Tabelle 3: Bezugszeichen der Fig. 2.**

| **Stoffstrom** | **Bedeutung** | **Apparat** | **Bedeutung** |
|---|---|---|---|
| **51** | Gasförmiges rohes Reaktionsprodukt (Rohanilin) | **2CC6** | Kreisgaskühler |
| **510** | Gasstrom aus **2V2** | **2V2** | Abscheider |
| **511** | Bodenablauf aus **2V2** | **2C3** | Kühler |
| **513** | Wässrige Phase aus **2D2** | **2D2** | Dekanter |
| **514** | Organische Phase aus **2D2** | **2H3** | Vorwärmer |
| **515** | Wässrige Phase aus **2D3** | **2T2** | Destillationskolonne |
| **516** | Organische Phase aus **2D3** | **2AT** | Abtriebsteil |
| **518** | Aus **2T2** über Kopf abgezogene Gasphase | **2VT1** | Unterer Verstärkungsteil |
| **519** | Kopfprodukt aus **2T2** nach Durchlaufen von **2CC3** | **2VT2** | Oberer Verstärkungsteil |
| **520** | Bodenablauf aus **2V3** | **2Hv1** | Verdampfer |
| **521** | Gasstrom aus **2V3** | **2C4** | Kühler |
| **522** | Anilinstrom aus Seitenentnahme von **2T2** | **2CC3** | Kondensator |
| **523** | Anilinstrom aus Seitenentnahme von **2T2** nach Durchlaufen von **2C4** | **2V3** | Abscheider |
| 524 | Umlauf zum Verdampfer **2Hv1** | **2D3** | Dekanter |
| **525** | Ausgeschleuster Bodenablauf aus **2T2** | **2CC6** | Kondensator |
| **528** | vereinigte Abwasserströme | | |
| **538** | rohes Reaktionsprodukt nach Durchlaufen von **2CC6** | | |
| **539** | Wässrige Baselösung | | |
| **540** | Vereinigte Ströme **514** und **539** | | |
| **541** | **540** nach Durchlaufen von **2H3** | | |

Folgende Bedingungen wurden zugrundegelegt:

| | |
|---|---|
| Temperatur des Gases nach **2CC6** (Strom **538):** | 60 °C |
| Zahl der theoretischen Stufen des oberen Verstärkungsteils von **2T2:** | 10 |
| Zahl der theoretischen Stufen des mittleren Verstärkungsteils von **2T2:** | 9 |
| Zahl der theoretischen Stufen des unteren Abtriebsteils von **2T2:** | 12 |

Bei dieser Verfahrensführung wird der Rohanilinstrom **51** nicht fraktionierend kondensiert, sondern im Kondensator **2CC6** in einem Schritt möglichst vollständig kondensiert (mehr als 95 % des Anilins). Das so erhaltene Verfahrensprodukt **(511** - das "Totalkondensat" dieses Verfahrens) wird zur Rückführung der nicht kondensierten Anteile in den Hydrierprozess (via Strom **510)** in einen Abscheider **2V2** geleitet. Das gesamte kondensierte Rohprodukt wird in **2C3** weiter gekühlt und dann einer Phasentrennung im Dekanter **2D2** unterworfen. Die Zuführung von Natronlauge **(539)** erfolgt zu der so erhaltenen organischen Phase **514.** Das nach Durchlauf einer geeigneten Mischeinrichtung erhaltene, NaOH-haltige Verfahrensprodukt **540** wird nach Durchlaufen eines Vorwärmers **2H3** zwischen **2AT** und **2VT1** in die Destillationskolonne **2T2** geleitet (Strom **541).** Der flüssige Ablauf aus dem oberen Verstärkungsteil **2VT2** wird vollständig als Seitenstrom (Strom **522)** entnommen und teilweise wieder als Rücklauf auf den mittleren Verstärkungsteil **2VT1** in die Kolonne zurückgeführt. Der nicht zurückgeführte Anteil wird in **2C4** gekühlt und als Reinanilinstrom **523** entnommen. Der Massenfluss des Sumpfproduktes, Strom **525,** wird so eingestellt, dass im Sumpf von **2T2** keine Phenolatsalze ausfallen. Die in **2T2** abgeführte Gasphase, Strom **518,** wird in **2CC3** unter Intertgaszugabe auf 40 °C gekühlt, bzw. kondensiert, und in eine Vorrichtung zur Trennung von Gasen und Flüssigkeiten **(2V3)** geleitet. Das Abgas (Strom **521)** wird der Verbrennung zugeführt. Die Flüssigphase von **2V3** wird im Dekanter **2D3** in eine anilinreiche und eine wasserreiche Phase getrennt. Die anilinreiche Phase wird als Rücklauf auf den Kopf der Kolonne **2T2** geführt **(516).**

Die Prozesswasserphasen aus **2D2** (Strom **513)** und **2D3** (Strom **515)** werden vereinigt (Strom **528)** und können zur Reinigung des Prozesswasserstroms und zur Rückgewinnung des enthaltenen Anilins z. B in einen Wasserstripper geleitet werden. Der gewonnene anilinreiche Strom kann z. B. in den Dekanter **2D3** zurückgeführt werden (in diesem Beispiel nicht mit betrachtet, da der energetische Aufwand für die Wasseraufbereitung in allen Fällen etwa gleich ist).

Die Ergebnisse sind in Tabelle 2 zusammengefasst:

**Tabelle 4: Zusammensetzungen der Ströme und nötiger Energieeintrag in Beispiel 1.**

| | **Strom^{[a]}** | | |
|---|---|---|---|
| | **510** | **523** | **525** |
| Gesamtmassenstrom | 13758 kg/h | 14725 kg/h | 320 kg/h |
| Nichtkondensierbare Gase | 64,8 % | < 1 ppm | < 1 ppm |
| Leichtsieder | 1,6 % | 127 ppm | < 1 ppm |
| Wasser | 28,4 % | 0,10 % | < 1 ppm |
| Anilin | 5,1 % | 99,9 % | 92,0 % |
| Summe aus Phenol und Natriumphenolat | 4 ppm | 14 ppm | 2,5 % |
| Hochsieder | < 1 ppm | < 1 ppm | 5,5 % |
| Temperatur | 60 °C | 40 °C | 150 °C |
| Druck | 3,1 bar (abs) | | |
| | | | |
| Für **2T2** nötiger Heizenergieeintrag | 0,286 kWh/kg Anilin im Produkt | | |

| | | | |
|---|---|---|---|
| [a] Gehaltsangaben in % und ppm sind stets Massenanteile. | | | |

Wie man sieht, involviert diese Vorgehensweise die erneute Verdampfung *des gesamten Anilins,* um es von den Hochsiedern abzutrennen. Auch müssen signifikante Anilinverluste über Strom **525** in Kauf genommen werden, um das Ausfallen von Phenolatsalzen in **2Hv1** zu vermeiden. Der gesamte für **2T2** erforderliche Energieeintrag ist mit 0,286 kWh/kg Anilin im Produkt sehr hoch.

### Beispiel 2 (erfindungsgemäß) ASPEN-Simulation

Diese Simulation wurde anhand der in Fig. 1 und weiter oben bereits näher erläuterten Verfahrensvariante durchgeführt. 5 % der in **1C2** kondensierten Flüssigkeit werden als Strom **15** in den Wäscher **1W1** geleitet. Folgende Bedingungen wurden zugrunde gelegt:

| | |
|---|---|
| Temperatur von Strom **10** nach **1C1:** | 120 °C |
| Temperatur des Gases nach **1C2** (Strom **17):** | 85 °C |
| Druck des Gases nach **1C2** (Strom 17): | 3,1 bar (abs) |

| | |
|---|---|
| Zahl der theoretischen Stufen in **1W1:** | 6 |
| Zahl der theoretischen Stufen von **1D1:** | 10 |
| Zahl der theoretischen Stufen von **1D2:** | 10 |
| Temperatur nach **1C3:** | 60 °C |

Die Ergebnisse sind in Tabelle 5 zusammengefasst.

**Tabelle 5: Zusammensetzungen der Ströme und nötiger Energieeintrag in Beispiel 2.**

| | **Strom^{[a]}** | | |
|---|---|---|---|
| | **40** | **30** | **60** |
| | **(Kreisgas)** | **(Reinanilin)** | **(Schwersieder)** |
| Gesamtmassenstrom | 13857 kg/h | 15113 kg/h | 40 kg/h |
| Nichtkondensierbare Gase | 64,4 % | < 1 ppm | < 1 ppm |
| Leichtsieder | 1,7 % | 338 ppm | < 1 ppm |
| Wasser | 28,7 % | 1,0 % | < 1 ppm |
| Anilin | 5,2 % | 99,0% | 56,0 % |
| Summe aus Phenol und Natriumphenolat | 2 ppm | 23 ppm | 0,3 % |
| Hochsieder | < 1 ppm | 1 ppm | 43,7 % |
| Temperatur | 60 °C | 101 °C | 160 °C |
| Druck | 3,1 bar (abs) | | |
| | | | |
| Für **1D1** nötiger Heizenergieeintrag | 0,022 kWh/kg Anilin im Produkt | | |
| Für **1D2** nötiger Heizenergieeintrag | 0,048 kWh/kg Anilin im Produkt | | |

| | | | |
|---|---|---|---|
| [a] Gehaltsangaben in % und ppm sind stets Massenanteile. | | | |

Wie man sieht, führt die Anwendung des erfindungsgemäßen Verfahrens zu einer deutlichen Reduktion sowohl des Energieverbrauchs als auch der Anilinverluste über Strom **225.** Der Gesamtheizenergieverbrauch in **1D1** und **1D2** beträgt nur noch 0,070 kWh/kg gegenüber 0,286 kWh/kg Anilin im Produkt im Vergleichsbeispiel. Die Produktqualität unterscheidet sich nur dadurch, dass Spuren an Phenolatsalzen im Produkt auftreten können, jedoch noch in akzeptabler Menge. Das Produkt enthält zwar darüber hinaus mehr Wasser als dasjenige des Vergleichsbeispiels 1. Wird das Produkt nachfolgend in einem Prozess mit Kondensationsreaktion (z. B. die Herstellung von Di- und Polyaminen der Diphenylmethanreihe) eingesetzt, ist der höhere Wassergehalt jedoch in der Regel nicht nachteilig. Falls gewünscht, kann der Wassergehalt auch durch eine höhere Verdampfungsrate in **1C9** auf ein niedrigeres Niveau reduziert werden. Hierin liegt im Übrigen auch ein weiterer Vorteil des Erfindungsgemäßen Verfahrens: Falls erforderlich ist es im Gegensatz zu der in PCT/EP2011/068122 (siehe Vergleichsbeispiele 3 und 4) beanspruchten Verfahrensweise ohne weiteren apparativen Aufwand möglich, ein nahezu wasserfreies Produkt zu erhalten. Um den Wassergehalt beispielsweise von 1 % in Beispiel 2 auf 0,1 % (vergleichbar mit den Vergleichsbeispielen 1 und 3) zu reduzieren, werden zusätzlich lediglich 0,037 kWh/kg Anilin an Heizenergie in **1C9** benötigt.

### Beispiel 3 (Vergleichsbeispiel) ASPEN-Simulation

Dieses Beispiel entspricht dem Verfahren gemäß PCT/EP2011/068122. Die Simulation greift das **Beispiel 3 des Dokuments auf, wobei jedoch aus Gründen der Vergleichbarkeit auf eine Betrachtung des Abwasserstrippers und damit auch auf die Einbindung des aus dem** Abwasserstrom 327 zurückgewonnenen anilinhaltigen Stroms verzichtet wird.

**Tabelle 6: Bezugszeichen der Fig. 3.**

| **Stoffstrom** | **Bedeutung** | **Apparat** | **Bedeutung** |
|---|---|---|---|
| **31** | Gasförmiges rohes Reaktionsprodukt (Rohanilin) | **3T1** | Wäscher |
| **32** | Aus **3T1** ausgetragenes flüssiges Verfahrensprodukt | **3CC1** | Kondensator |
| **33** | Gasstrom aus **3T1** | **3V1** | Abscheider |
| **34** | 33 nach Durchlaufen von **3CC1** | **3S1** | Splitter |
| **35** | In den Kopf von **3T1** zurückgeführter Produktstrom | **3C2** | Kühler |
| **351** | In **3S1** abgezweigter Produktstrom, der nicht in **3T1** zurückgeführt wird | **3CC2** | Kondensator |
| **36** | **32** nach Durchlaufen von **3C1** | **3V2** | Abscheider |
| **361** | **36** nach Vermischung mit **313** | **3C3** | Kühler |
| **37** | Wässrige Baselösung | **3D2** | Dekanter |
| **38** | Kopfprodukt aus **3V1** | **3C1** | Kühler |
| **39** | **38** nach Durchlaufen von **3CC2** | **3D1** | Dekanter |
| **310** | Gasstrom aus **3V2** | **3H1** | Vorwärmer |
| **311** | Aus **3V2** ausgetragenes flüssiges Verfahrensprodukt | **3D3** | Dekanter |
| **3111** | **311** nach Vermischung mit dem Strom aus **3C2** | **3T2** | Destillationskolonne |
| **312** | Wässrige Baselösung | **3AT** | Abtriebsteil |
| **313** | Wässrige Phase aus **3D2** | **3VT1** | Unterer Verstärkungsteil |
| **314** | Organische Phase aus **3D2** | **3VT2** | Oberer Verstärkungsteil |
| **315** | Wässrige Phase aus **3D3** | **3Hv1** | Verdampfer |
| **316** | Organische Phase aus **3D3** | **3C4** | Kühler |
| **317** | Organische Phase aus **3D1** | **3CC3** | Kondensator |
| **318** | Aus **3T2** über Kopf abgezogene Gasphase | **3V3** | Abscheider |
| **319** | **318** nach Durchlaufen von **3CC3** | | |
| **320** | Aus **3V3** unten abgezogener flüssiger Kondensatstrom | | |
| **321** | Gasstrom aus **3V3** | | |
| **322** | Anilinstrom aus Seitenentnahme von **3T2** | | |
| **323** | Anilinstrom aus Seitenentnahme von **3T2** nach Durchlaufen von **3C4** | | |
| **324** | Umlauf zum Verdampfer **3Hv1** | | |
| **325** | Ausgeschleuster Bodenablauf aus **3T2** | | |
| **326** | Wässrige Phase aus **3D1** | | |
| **9** | Flüssiger Strom aus **3V1** | | |
| **11** | **351** nach Durchlaufen von **3C2** | | |

Folgende Bedingungen wurden zugrunde gelegt:

| | |
|---|---|
| Temperatur des Gases nach **3CC1** (Strom **34):** | 85 °C |
| Druck des Gases nach **3CC1** (Strom **34):** | 3,1 bar (abs) |
| Zahl der theoretischen Stufen in **3T1:** | 6 |
| Zahl der theoretischen Stufen von **3AT:** | 12 |
| Zahl der theoretischen Stufen von **3VT1:** | 9 |
| Zahl der theoretischen Stufen von **3VT2:** | 10 |
| Temperatur nach **3CC2:** | 60 °C |

Die Ergebnisse sind in Tabelle 6 zusammengefasst.

**Tabelle 7: Zusammensetzungen der Ströme und nötiger Energieeintrag in Beispiel 3.**

| | **Strom^{[a]}** | | |
|---|---|---|---|
| | **310** | **323** | **325** |
| | **(Kreisgas)** | **(Reinanilin)** | **(Schwersieder)** |
| Gesamtmassenstrom | 13857 kg/h | 15076 kg/h | 40 kg/h |
| Nichtkondensierbare Gase | 64,4 % | < 1 ppm | < 1 ppm |
| Leichtsieder | 1,7 % | 352 ppm | < 1 ppm |
| Wasser | 28,7 % | 0,7 % | < 1 ppm |
| Anilin | 5,2 % | 99,2 % | 56,1 % |
| Summe aus Phenol und Natriumphenolat | 2 ppm | 19 ppm | 0,1 % |
| Hochsieder | < 1 ppm | 1 ppm | 43,7 % |
| Temperatur | 60 °C | 40 °C | 161 °C |
| Druck | 3,1 bar (abs) | | |
| | | | |
| Für **3Hv1** nötiger Heizenergieeintrag | 0,096 kWh/kg Anilin im Produkt | | |

| | | | |
|---|---|---|---|
| [a] Gehaltsangaben in % und ppm sind stets Massenanteile. | | | |

Der Heizenergiebedarf in **3Hv1** ist mit 0,096 kWh/kg Anilin im Produkt etwa 40 % höher als im erfindungsgemäßen Beispiel 2. Zusätzlich muss für den Prozess nach Beispiel 3 der Heizenergieeintrag auf hohem Temperaturniveau bzw. mit hochgespanntem Dampf einbracht werden, während im erfindungsgemäßen Beispiel 2 mehr als 50 % der gesamten Heizenergie (in Apparat **3C9)** auf niedrigerem Temperaturniveau bzw. mit Hilfe von minderwertigem Dampf niedriger Druckstufe eingebracht werden kann. Darüber hinaus ist der apparative Aufwand im Beispiel 3 wesentlich größer, da die basische Wäsche für zwei Ströme separat erfolgen muss.

### Beispiel 4 (Vergleichsbeispiel) ASPEN-Simulation

Dieses Beispiel entspricht dem Verfahren gemäß PCT/EP2011/068122. Bei der Berechnung des Energiebedarfs wurde zusätzlich eine Produkttrocknung in einer Strippkolonne **(4T4)** auf einen Restgehalt an Wasser von 1 Massen-% berücksichtigt.

**Tabelle 8: Bezugszeichen der Fig. 4.**

| **Stoffstrom** | **Bedeutung** | **Apparat** | **Bedeutung** |
|---|---|---|---|
| **41** | Gasförmiges rohes Reaktionsprodukt (Rohanilin) | **4T1** | Wäscher |
| **42** | Aus **4T1** ausgetragenes flüssiges Verfahrensprodukt | **4CC1** | Kondensator |
| **43** | Gasstrom aus **4T1** | **4V1** | Abscheider |
| **44** | **43** nach Durchlaufen von **4CC1** | **4S1** | Splitter |
| **45** | In den Kopf von **4T1** zurückgeführter Produktstrom | **4C2** | Kühler |
| **451** | In **4S1** abgezweigter Produktstrom, der nicht in **4T1** zurückgeführt wird | **4CC2** | Kondensator |
| **48** | Gasstrom aus **4V1** | **4V2** | Abscheider |
| **49** | **48** nach Durchlaufen von **4CC2** | **4C3** | Kühler |
| **410** | Gasstrom aus **4V2** | **4D2** | Dekanter |
| **411** | Aus **4V2** ausgetragenes flüssiges Verfahrensprodukt | **4T2** | Destillationskolonne |
| **4111** | **411** nach Vermischung mit dem Strom aus **4C2** | **4AT** | Abtriebsteil |
| **41111** | **4111** nach Vermischung mit **420** | **4VT1** | Unterer Verstärkungsteil |
| **413** | Wässrige Phase aus **4D2** | **4VT2** | Oberer Verstärkungsteil |
| **414** | Organische Phase aus **4D2** | **4Hv1** | Verdampfer |
| **418** | Aus **4T2** über Kopf abgezogene Gasphase | **4V3** | Abscheider |
| **420** | Aus **4V3** unten abgezogener flüssiger Kondensatstrom | **4C5** | Kühler |
| **421** | Gasstrom aus **4V3** | **4D4** | Dekanter |
| **422** | Anilinstrom aus Seitenentnahme von **4T2** | **4D5** | Dekanter |
| **424** | Umlaufstrom zum Verdampfer **4Hv1** | **4CC4** | Kondensator |
| **425** | Ausgeschleuster Bodenablauf aus **4T2** | **4T3** | Wasserstripper |
| **428** | Kopfprodukt aus **4T3** (Anilin-Wasser-Azeotrop) | **4Hv2** | Verdampfer |
| **429** | Umlaufstrom zum Verdampfer **4Hv2** | **4C7** | Kühler |
| **430** | Ausgetragener Bodenablauf aus **4T3** | **4C8** | Wärmeaustauscher |
| **432** | Wässrige Baselösung | **4T4** | Strippkolonne zur Trocknung von **434** |
| **433** | Organische Phase aus **4D4** | **4C9** | Verdampfer |
| **434** | Organische Phase aus **4D5** (= wasserhaltiges gereinigtes Anilin) | | |
| **435** | Wässrige Phase aus **4D4** | | |
| **436** | Wässrige Phase aus **4D5** | | |
| **480** | Gasstrom aus **4T4** | | |
| **481** | Ausgeschleuster Bodenablauf aus **4T4** (getrockneter Anilinstrom) | | |
| **Q** | Wärmeintegration zwischen **4C7** und **4C8** | | |

Folgende Bedingungen wurden zugrunde gelegt:

| | |
|---|---|
| Temperatur des Gases nach **4CC1** (Strom **44):** | 85 °C |
| Druck des Gases nach **4CC1** (Strom **44):** | 3,1 bar (abs) |
| Zahl der theoretischen Stufen in **4T1:** | 6 |
| Zahl der theoretischen Stufen von **4AT:** | 12 |
| Zahl der theoretischen Stufen von **4VT1:** | 9 |
| Zahl der theoretischen Stufen von **4VT2:** | 10 |
| Zahl der theoretischen Stufen von **4T3:** | 30 |
| Zahl der theoretischen Stufen von **4T4:** | 10 |
| Temperatur nach **4CC2:** | 60 °C |

Die Ergebnisse sind in Tabelle 9 zusammengefasst.

**Tabelle 9: Zusammensetzungen der Ströme und nötiger Energieeintrag in Beispiel 4.**

| | **Strom^{[a]}** | | |
|---|---|---|---|
| | **410** | **481** | **425** |
| | **(Kreisgas)** | **(Reinanilin)** | **(Schwersieder)** |
| Gesamtmassenstrom | 13857 kg/h | 15151 kg/h | 40 kg/h |
| Nichtkondensierbare Gase | 64,4 % | < 1 ppm | < 1 ppm |
| Leichtsieder | 1,7 % | 329 ppm | < 1 ppm |
| Wasser | 28,7 % | 1,0 % | < 1 ppm |
| Anilin | 5,2 % | 99,0 % | 53,8 % |
| Summe aus Phenol und Natriumphenolat | 2 ppm | 23 ppm | 2,5 % |
| Hochsieder | < 1 ppm | 1 ppm | 43,7 % |
| Temperatur | 60 °C | 119 °C | 161 °C |
| Druck | 3,1 bar (abs) | | |
| | | | |
| Für 4Hv1 nötiger Heizenergieeintrag | 0,070 kWh/kg Anilin im Produkt | | |
| Für 4C9 nötiger Heizenergieeintrag | 0,044 kWh/kg Anilin im Produkt | | |

| | | | |
|---|---|---|---|
| [a] Gehaltsangaben in % und ppm sind stets Massenanteile. | | | |

Zwar muss nach Beispiel 4 wie im erfindungsgemäßen Beispiel 2 nur ein Strom einer basischen Wäsche unterzogen werden. Der Heizenergiebedarf ist mit 0,114 kWh/kg Anilin im Produkt jedoch etwa 60 % höher als im erfindungsgemäßen Beispiel 2.

## Patentansprüche

1. Verfahren zur Herstellung von Anilin umfassend die folgenden Schritte:
(i) Gasphasenhydrierung von Nitrobenzol in Gegenwart eines Katalysators,
(ii) fraktionierende Kondensation des in (i) anfallenden gasförmigen Rohproduktes in n Kondensationsstufen, wobei n eine natürliche Zahl von 2 bis 8 ist, mit sukzessive sinkender Kondensationstemperatur, wobei in der ersten bis (n-1)ten Kondensationsstufe jeweils ein flüssiges Partialkondensat PKⁱ (PK¹, PK², ... PKⁿ⁻¹) und in der nten Kondensationsstufe ein flüssiges Totalkondensat (TK) erhalten wird,
(iii)
falls (a) n = 2 ist, Destillation des einen in (ii) erhaltenen flüssigen Partialkondensats PK¹,
falls (b) n ≥ 3 ist, Destillation eines Teils der in (ii) erhaltenen flüssigen Partialkondensate PKⁱ,
wobei ein Destillat PKD erhalten wird,
(iv) Vereinigung
(a) sofern vorhanden, wenigstens eines Teils der in (ii) erhaltenen und nicht in (iii) destillierten Partialkondensate PKⁱ,
(b) des in (iii) erhaltenen Destillats PKD und
(c) mindestens des organischen Anteils des Totalkondensats TK,
und Extraktion der so gewonnenen Produktmischung mit wässriger Baselösung und Trennung des so erhaltenen Gemischs in eine wässrige Phase und in eine organische, Anilin umfassende Phase.

2. Verfahren nach Anspruch 1, bei dem das Totalkondensat vor Durchführung von Schritt (iv) in eine organische Phase (TKO) und eine wässrige Phase (TKW) getrennt und (c) nur die organische Phase TKO mit (a) sofern vorhanden, wenigstens einem Teil der in (ii) erhaltenen und nicht in (iii) destillierten Partialkondensate PKⁱ und (b) dem in (iii) erhaltenen Destillat PKD vereinigt wird.

3. Verfahren nach Anspruch 1 oder 2, bei dem Schritt (ii) genau drei Kondensationsstufen umfasst.

4. Verfahren nach einem der Ansprüche 1 bis 3, bei dem die erste Kondensationsstufe bei einer Temperatur T¹ von 100 °C bis 200 °C, die nte Kondensationsstufe bei einer Temperatur Tⁿ von 15 °C bis 90 °C betrieben wird, und die Temperatur jeder weiteren Kondensationsstufe, sofern vorhanden, jeweils um 1 K bis 100 K unter der vorangehenden Kondensationsstufe liegt.

5. Verfahren nach Anspruch 1, bei dem die in (iv) erhaltene organische, Anilin umfassende Phase
(v) mit einem mindestens 85 Massen-% Wasser umfassenden Strom, bezogen auf die Gesamtmasse dieses Stroms, gewaschen wird.

6. Verfahren nach Anspruch 5, bei dem der mindestens 85 Massen-% Wasser umfassende Strom durch Kondensation von wasserhaltigen Dämpfen erzeugt wurde.

7. Verfahren nach einem der Ansprüche 2 bis 4, bei dem die in (iv) erhaltene organische, Anilin umfassende Phase
(v) mit einem mindestens 85 Massen-% Wasser umfassenden Strom, bezogen auf die Gesamtmasse dieses Stroms, gewaschen wird.

8. Verfahren nach Anspruch 7, bei dem der mindestens 85 Massen-% Wasser umfassende Strom die aus dem nten Kondensat TK abgetrennte wässrige Phase TKW oder ein Teil davon ist.

9. Verfahren nach einem der Ansprüche 1 bis 4, bei dem die in (iv) erhaltene organische, Anilin umfassende Phase gestrippt wird.

10. Verfahren nach einem der Ansprüche 5 bis 8, bei dem die in (v) erhaltene gewaschene organische, Anilin umfassende Phase gestrippt wird.

11. Verfahren nach einem der Ansprüche 1 bis 10, bei dem in Schritt (iv) als wässrige Baselösung eine Lösung eines Alkalimetallhydroxids oder Erdalkalimetallhydroxids oder eines Gemisches aus Alkalimetallhydroxid und Erdalkalimetallhydroxid in Wasser eingesetzt wird.

12. Verfahren nach einem der Ansprüche 1 bis 11, bei dem alle anfallenden wässrigen Ströme vereinigt werden und der vereinigte Abwasserstrom einer Strippung unterzogen wird, um Anilin als Azeotrop mit Wasser zurückzugewinnen.
